(19) Europäisches Patentamt
European Patent Office
Office européen des brevets

(11) **EP 1 008 854 B1**

(12) **FASCICULE DE BREVET EUROPEEN**

(45) Date de publication et mention
de la délivrance du brevet:
**10.03.2004 Bulletin 2004/11**

(51) Int Cl.⁷: **G01N 33/96**, G01N 33/569

(21) Numéro de dépôt: **99870255.9**

(22) Date de dépôt: **07.12.1999**

(54) **Standard basé sur un pool de plasmas sanguins de plus de 200 patients sains**

Plasmasammlungen von mehr als 200 gesunden Patienten und davon abgeleiteter Kalibrierstandard

Calibrant based on a plasma pool from more than 200 healthy patients

(84) Etats contractants désignés:
**AT BE CH CY DE DK ES FI FR GB GR IE IT LI LU MC NL PT SE**

(30) Priorité: **07.12.1998 BE 9800882**

(43) Date de publication de la demande:
**14.06.2000 Bulletin 2000/24**

(73) Titulaires:
• **S.C.R.L. Departement Central de Fractionnement de la Croix-Rouge**
**1120 Bruxelles (BE)**
• **UNIVERSITE LIBRE DE BRUXELLES**
**1050 Bruxelles (BE)**

(72) Inventeurs:
• **Laub, Ruth**
**1190 Bruxelles (BE)**
• **Duchateau, Jean**
**7060 Horrues (BE)**
• **Giambattista, Mario**
**7090 Braine-le-Comte (BE)**

(74) Mandataire: **Van Malderen, Michel et al**
**Office van Malderen**
**Place Reine Fabiola 6/1**
**1083 Bruxelles (BE)**

(56) Documents cités:
EP-A- 0 787 989      US-A- 5 798 267

• **SASAKI T ET AL: "Cross-reactive antibodies to mycoplasmas found in human sera by the enzyme-linked immunosorbent assay ( ELISA )." MICROBIOLOGY AND IMMUNOLOGY, (1987) 31 (6) 521-30. , XP000892669**

• **KAO K J ET AL: "Enzyme-linked immunoassay for anti-HLA antibodies--an alternative to panel studies by lymphocytotoxicity." TRANSPLANTATION, (1993 JAN) 55 (1) 192-6. , XP000892675**

• **QUATAERT SA, KIRCH CS, WIEDL LJ, PHIPPS DC, STROHMEYER S, CIMINO CO, SKUSE J, MADORE DV: "Assignment of weight-based antibody units to a human antipneumococcal standard reference serum, lot 89-S" CLINICAL AND DIAGNOSTIC LABORATORY IMMUNOLOGY, vol. 2, no. 5, septembre 1995 (1995-09), pages 590-597, XP000892694**

• **LIU P ET AL: "A prospective study of a serum - pooling strategy in screening blood donors for antibody to hepatitis C virus." TRANSFUSION, (1997 JUL) 37 (7) 732-6. , XP000892639**

• **HAZLEWOOD M; NUSRAT R; KUMARARATNE D S; GOODALL M; RAYKUNDALIA C; DA DONG WANG; JOYCE H J; MILFORD-WARD A; FORTE M; PAHOR A: "The acquisition of anti-pneumococcal capsular polysaccharide Haemophilus influenzae type b and tetanus toxoid antibodies, with age, in the UK" CLINICAL AND EXPERIMENTAL IMMUNOLOGY, vol. 93, 1993, pages 157-164, XP000892905**

• **MAHONEY R J ET AL: "Inhibition of HLA antibody cytotoxicity by intravenous immunoglobulin G F(ab')2 dimers, monomers, and monovalent F(ab)." HUMAN IMMUNOLOGY, (1999 JUN) 60 (6) 492-9. , XP000892666**

Remarques:
Le dossier contient des informations techniques présentées postérieurement au dépôt de la demande et ne figurant pas dans le présent fascicule.

## Description

### Objet de l'invention

La présente invention se rapporte à un standard incorporé dans des tests de diagnostic et/ou de quantification d'anticorps résultant d'une infection par un agent bactérien ou viral ou résultant d'une réaction auto-immune ou allergique.

### Etat de la technique et arrière-plan technologique à la base de l'invention

Les matériaux et standards de référence dans le domaine biologique sont des préparations de substances ayant une composition moléculaire complexe intervenant dans les mécanismes de contrôle de qualité, de diagnostic et de recherche.

Pour le diagnostic des agents bactériens responsables de différentes infections affectant des mammifères, y compris l'homme, il a été proposé différentes méthodes pour obtenir une référence standardisée dans un tel diagnostic.

Les organismes de remboursement de frais médicaux (INAMI) spécifient qu'une quantification d'immunoglobulines, en particulier la quantification d'une hypogammaglobulinémie, est caractérisée lorsque la teneur totale en immunoglobulines est inférieure à la valeur de norme du laboratoire. Cette valeur de norme doit être calculée comme deux déviations standard en dessous de la moyenne mesurable liée à des contrôles par tranche d'âge ou de 95% de l'intervalle de confiance d'une population de contrôle couplé à l'âge. Par conséquent, la caractérisation dans un test de diagnostic ou de quantification d'un certain type d'anticorps éventuellement dirigés contre un agent bactérien, doit se faire par rapport à un standard qui est constitué de la valeur de la norme du laboratoire. Par conséquent, ces normes varient très fort d'un laboratoire à un autre, ce qui peut fausser les résultats.

Quataert et al. ((*Clinical and Diagnostic Laboratory Immunology* **Vol. 2 No. 5**, pp. 590-597 (Septembre 1995)) décrivent une méthode d'identification des anticorps spécifiques à chacun des sérotypes de *S. pneumoniae*. Cependant, le lot de référence standard proposé est obtenu à partir d'un pool de 17 adultes vaccinés avec une préparation vaccinale, qui peuvent présenter entre eux une trop grande variance, et nécessitera l'utilisation d'extrapolations mathématiques particulièrement complexes pour le diagnostic de cette infection bactérienne. De plus, une cross-réactivité entre les divers antigènes capsulaires des différents sérotypes peut se présenter (Coughlin et al., *Vaccine 16*, N°18, p. 1761-1767, 1998).

EP 0 787 989 et US 5 798 267 montrent la préparation d'un standard par "pooling" de respectivement 1000 et 1200 plasmas. Ces documents ne concernent pas des anticorps.

### Buts de l'invention

La présente invention vise à fournir un standard amélioré dans des dispositifs de diagnostic, qui ne présenterait pas les inconvénients de l'état de la technique, et qui puisse être utilisé pour améliorer le diagnostic, la quantification et/ou le monitoring de réactions immunitaires résultant d'infections affectant des mammifères, y compris l'homme, en particulier résultant d'infections par un agent bactérien ou viral ou résultant d'une réaction auto-immune ou allergique.

Un but particulier de la présente invention est de fournir un standard dans un tel dispositif de diagnostic, qui serait d'une conception simple et peu coûteuse et qui permettrait de simplifier le diagnostic de telles infections sans nécessiter l'utilisation de modifications des protocoles opératoires des diagnostics habituellement utilisés, et plus particulièrement qui ne nécessiterait pas l'utilisation d'extrapolations mathématiques complexes.

### Eléments caractéristiques de l'invention

La présente invention concerne un dispositif de diagnostic et/ou de quantification (y compris de monitoring ou de suivi) d'une réaction immunitaire caractérisée par la présence d'un certain type d'anticorps, qui peut être liée à une infection telle qu'une infection bactérienne ou virale ou une réaction de type auto-immune ou allergique, en particulier une allergie alimentaire, susceptible d'affecter un patient, en particulier un mammifère (y compris l'homme).

Le dispositif de l'invention permet la détection et/ou la quantification des anticorps présents dans un liquide biologique extrait dudit patient tel qu'un échantillon sanguin dudit patient, lesdits anticorps étant dirigés contre l'agent responsable de l'infection ou des portions de celui-ci (en particulier le polysaccharide C présent sur la paroi des bactéries) ou étant significatifs d'une réaction immunitaire ou allergique, le dispositif de l'invention étant caractérisé en ce qu'il comprend, en tant que standard (dénommé également ci-après "référence" ou "calibrateur"), un échantillon biologique comprenant un titre défini d'anticorps sélectionné à partir d'un pool (c'est-à-dire un mélange) de plasmas sanguins obtenus à partir de plus de 200 patients ou individus sains.

On entend par "patient", tout mammifère, y compris l'homme, susceptible d'être infecté par ledit microorganisme responsable de cette infection ou présentant une réaction allergique ou auto-immune, et susceptible de produire des anticorps dirigés spécifiquement contre ledit microorganisme ou susceptible de présenter des anticorps liés à cette réaction auto-immune ou allergique.

On entend par "patient (ou individu) sain", un patient ayant été en contact avec ledit microorganisme responsable de ladite infection et ne présentant pas ou ne présentant plus ladite infection. Par exemple, on entendrait par "patients (ou individus) sains", des adultes sains d'une population exposée, même de manière transitoire, à une infection bactérienne, en particulier par *Streptococcus pneumoneia*, et possédant des anticorps de type anti-*Streptococcus pneumoniae*. Par conséquent, un mélange de plasmas ou de sérums de patients (ou d'individus) sains, c'est-à-dire ne présentant pas ou ayant une faible probabilité de présenter ladite infection, peut servir de standard.

Les Inventeurs ont remarqué que si l'échantillon biologique préparé à partir d'un tel pool, c'est-à-dire un mélange de plasmas, réalisé à partir de plus de 200 patients, de préférence lorsque le nombre de patients, excède 1000, 2000, voire 5000 patients sains, sera caractérisé par une très faible variance (statistique) et pourra servir de standard "universel" pour des dispositifs de diagnostic et/ou de quantification, car ils seront caractérisés par une très faible variance même si dans ledit pool de plasmas, un ou plusieurs des patients ou individus sains présentaient, au moment du don de sang, ladite infection que l'on cherche à caractériser. En effet, le titre de ces anticorps par rapport au nombre d'anticorps sélectionnés à partir d'autres adultes caractérisés en tant que patients ou individus sains, n'affecterait pas de manière significative les propriétés avantageuses du standard de l'invention.

En outre, le standard ou calibrateur de l'invention peut être avantageusement préparé à partir d'un cryo-surnageant dudit pool de plasmas, qui peut être disponible aisément en grande quantité.

Un tel standard permet non seulement un diagnostic mais également une quantification (calcul du titre d'anticorps) ainsi que le monitoring (dénommé également "suivi") de ladite réaction immunitaire. Ce monitoring est particulièrement utile lorsque l'on souhaite voir l'évolution d'une vaccination vis-à-vis d'un microorganisme responsable d'une infection, ou vis-à-vis d'un traitement anti-allergique ou destiné au traitement d'une pathologie ou d'un syndrome auto-immunitaire.

Le standard ou référence de l'invention, qui sera constitué d'un titre défini d'anticorps caractérisé par une faible variance, c'est-à-dire par une différence significative entre les types d'anticorps, permet l'utilisation aisée d'un tel standard dans un dispositif de diagnostic et/ou de quantification sans nécessiter d'extrapolations ni d'analyses complexes mathématiques et sans affecter les procédures habituelles de diagnostic, de quantification et éventuellement de monitoring susmentionnées. Un tel standard peut être avantageusement utilisé dans tout type de dispositif de diagnostic ou de quantification, en particulier dans tout type de trousse de diagnostic et/ou de quantification basé sur les technologies ELISA telles que décrites ci-dessous ou d'autres technologies de détection d'anticorps telles que les technologies RIA ou d'hémagglutination, et sur tout type de support.

Le dispositif et le procédé de l'invention peuvent également inclure un milieu et une étape permettant une incubation préalable des échantillons à tester, de préférence en présence de polysaccharide C, avantageusement d'environ 2,5 $\mu$M/ml de polysacharide C pendant une durée de 30 min. à 37°C, pour doser les anticorps capsulaires dans les différentes préparations d'un test immunochimique en utilisant comme antigènes immobilisés des préparations d'antigènes polysaccharidiques (par exemple pour le diagnostic du *Streptococcus pneumoniae*).

Le dispositif de diagnostic de l'invention est particulièrement adapté pour le diagnostic, la quantification et/ou le monitoring de réactions immunitaires liées à une réaction allergique, en particulier pour la détection, la quantification et/ou le monitoring des anticorps de type IgG ou IgE liés à une réaction allergique, en particulier des réactions allergiques alimentaires, pour la détection d'auto-anticorps dans le cas d'une réaction auto-immune ou pour la détection, la quantification et/ou le monitoring des anticorps dirigés contre différentes infections bactériennes ou virales, en particulier des infections par des souches de *Streptococcus pneumoniae*, de *Neiseria* et d'*Hémophilus*.

L'objet de l'invention sera décrit de manière détaillée dans l'exemple d'exécution ci-dessous.

Figures

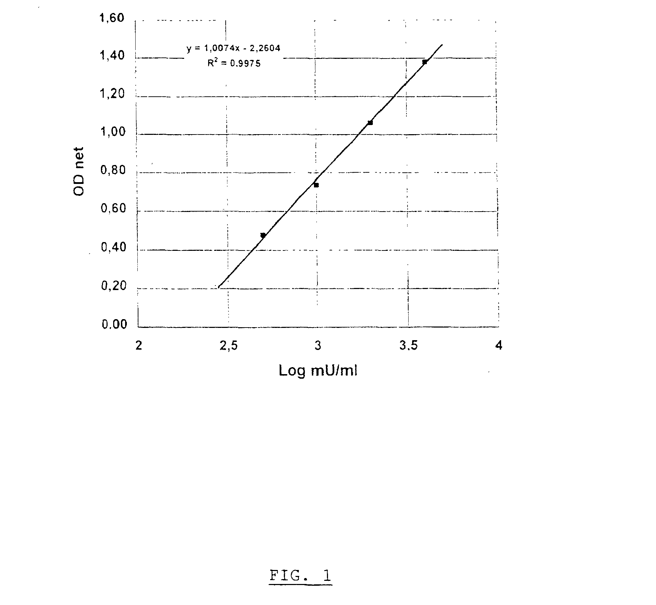

La figure 1 représente la droite de calibration.

**Exemple 1 : Test immuno-enzymatique ELISA pour la détection d'anticorps anti-pneumocoques dans le plasma, les pools de plasma et les concentrés d'immunoglobulines**

La souche *Streptococcus pneumonia* est un hôte commun de la flore oro-pharyngée, dont la capsule confère la virulence et l'antigénéicité. Les anticorps dirigés contre les antigènes polysaccharidiques de la capsule sont protecteurs de l'infection. Le taux des porteurs de *Streptococcus pneumoneia* varie selon l'âge, la saison et l'environnement infectieux ou non. Les pneumocoques sont la cause majeure des pneumonies acquises dans les communautés et la source fréquente d'otites aiguës de l'oreille moyenne, de sinusites et de pharyngites. Les pneumonies, qui sont provoquées dans la majorité des cas par des pneumocoques, sont rapportées comme étant la cinquième cause de décès aux Etats-Unis. En Belgique, l'incidence des infections par pneumocoques est de 200.000 cas par an. Le taux des souches antibiorésistantes croît constamment, 12% étant résistantes à la tétracycline et 21% à l'érythromycine. Le taux de mortalité est estimé à 2.000 décès par an, qui semblent liés à des problèmes d'antibiorésistance.

Si les pneumocoques touchent tous les individus (70% des porteurs sains), certains terrains prédisposent à l'infection pneumococcique comme l'âge, les broncho-pneumopathies, les cancers des voies séro-digestive supérieures, l'éthylisme et le tabagisme, ainsi que l'insuffisance cardiaque. Les hypo-asplénies représentent un facteur aggravant certain ainsi que les hémopathies touchant en particulier les enfants âgés de moins de 5 ans.

La recherche des anticorps anti-pneumocoques dans le plasma et les fractions dérivées par la trousse de diagnostic de l'invention permet de détecter les anticorps anti-polysaccharidiques de la capsule des 23 sérotypes les plus fréquents (95% des souches isolées en Belgique). Le test immuno-enzymatique ELISA décrit ci-dessous permet le dosage quantitatif des anticorps anti-pneumocoques dans un plasma ou une fraction plasmatique sans traitement préalable de l'échantillon plasmatique récolté sur anticoagulants. Celui-ci permet de détecter les anticorps dans les plasmas ou fractions plasmatiques contenant peu d'anticorps (concentration 1000 fois moindre que la moyenne rencontrée chez les individus sains) ainsi que dans les plasmas hyperhumains obtenus après vaccination.

Le dispositif de l'invention permet donc un monitoring (ou suivi) particulièrement efficace d'une vaccination ou de l'évolution d'une infection.

### *Préparation des solutions, dilutions et échantillons*

Solution A : Tampon PBS 10x concentré

- 400 g NaCl
- 10 g KCl
- 72 g $Na_2HPO_4$
- 12 g $KH_2PO_4$

Porter à 5 litres avec de l'eau mQ

Solution B : Solution de lavage (PBS 1x-Tween® 0,1%)

Les concentrations finales sont respectivement de :

- 0,14 M NaCl
- 2,7 mM KCl
- 10 mM $Na_2HPO_4$
- 1,8 mM $KH_2PO_4$
- 0,1% Tween® 20

Pour 1000 ml de solution de travail, prendre :

- 100 ml de solution A

- 1 ml de Tween® 20

- 899 ml d'eau mQ.

Solution C : Tampon de dilution de l'anticorps marqué à la peroxydase

*Solution $C_0$ :* solution stock d'albumine bovine à 5% Pour 10 ml de solution :

- 500 mg de BSA (Sigma)

Porter à 10 ml final avec de l'eau mQ. La solution est stockée à 20 °C.

*Solution C de travail :*

Pour 10 ml de solution C :

- 1 ml de solution $C_0$
- 1 ml de solution A
- 8 ml d'eau mQ

Solution D : Solution de saturation et de dilution des échantillons à doser

- dissoudre 20 g de caséine (Merck) dans 400 ml d'eau mQ
- chauffer jusqu'à 60 °C et ajuster régulièrement le pH à 9.6 avec NaOH 5 M
- mélanger en chauffant à 60 °C pendant 2 h sur agitateur magnétique
- ajouter 36 g de NaCl et 0,8 g de Thimérosal® (Merck)
- ajouter lentement 1600 ml d'eau mQ et 200 ml de Tris-HCl 20 mM, pH 6,6 (Merck)
- ajuster le pH à 7,2 - 7,4 avec HCl 1 M ou NaOH 1 M
- centrifuger la solution à 4500 rpm pendant 1 h à 22 °C
- laisser décanter et filtrer sur Wattman®
- congeler à -20 °C dans des flacons de 500 ml
- filtrer avant utilisation sur Milli-Fil PF® 0,8 µm (Millipore) et conserver à 4 °C

La solution finale est de 10 mM tris; pH 7,2 - 7,4; 0,15 M NaCl; 0,02 % Thimérosal®; 0,5% caséine

Solution E : Solution d'arrêt de la réaction

- $H_2SO_4$ : 2 N

Préparation des dilutions du calibrateur

Un flacon de calibrateur est reconstitué dans 1 ml de caséine "prête à l'emploi" (calibrateur 1/1).

Exemples :

*Calibrateur à 1/125 :* 16 µl de calibrateur 1/1 + 1984 µl de caséine "prête à l'emploi"
*Calibrateur à 1/250 :* 400 µl de calibrateur 1/125 + 400 µl de caséine "prête à l'emploi"
*Calibrateur à 1/500 :* 400 µl de calibrateur 1/250 + 400 µl de caséine "prête à l'emploi"
*Calibrateur à 1/1000 :* 400 µl de calibrateur 1/500 + 400 µl de caséine "prête à l'emploi"

Préparation des différents échantillons à tester

Exemples :

*Echantillon n° 1 à 1/250 :* prélever 8 µl d'échantillon à tester + 1992 µl de caséine
*Echantillon n° 2 à 1/500 :* prélever 400 µl de l'échantillon n° 1 + 400 µl de caséine

***Procédure***

A. Préparation de la solution Pneumovax® R[23] pour le "coating"

La concentration en antigène du vaccin Pneumovax® R[23] (Pasteur Mérieux MSD) est de 1,15 mg/ml. La concentration de travail est de 1 µg/ml.

Pour une plaque (96 puits) :

- 9,85 ml d'eau mQ
- 1,15 ml de solution A
- 11 ml de solution stock de Pneumovax® R [23]

Ajouter à l'aide d'une multipipette 100 µl de la solution d'antigène à 1 µg/ml dans chaque puits.

Recouvrir la plaque d'un adhésif transparent.

Incuber 16 h à 4 °C.

### B. Lavage de la plaque

Laver 5 fois la microplaque avec la solution B à l'aide d'un laveur de plaques (NUNC® Immuno Wash 8).

Secouer la plaque sur un papier absorbant de manière à la sécher au maximum.

### C. Saturation des sites non spécifiques

Ajouter 200 µl de la solution D dans chaque puits à l'aide d'une multipipette.

Recouvrir la microplaque d'un adhésif transparent.

Incuber 30 min à température ambiante.

Eliminer la solution et sécher au maximum la plaque en la secouant sur un papier absorbant.

### D. Incubation avec l'échantillon à tester

Pendant l'étape de saturation (étape C), préparer les dilutions des différents échantillons à tester.

*D-1 : Si l'échantillon à tester est un plasma :*

Toutes les dilutions des échantillons à tester se font dans la solution D.

Pour obtenir une courbe de calibration, il faut :

- reconstituer le plasma pool standard avec 1 ml d'eau mQ (mélanger 2 min au vortex)
- préparer 250 µl de plasma du pool standard dilué à 1/125; 1/250; 1/500 et 1/1000 dans la solution D comme décrit ci-dessus

Pour préparer les échantillons à tester, il faut :

- préparer 250 µl d'une dilution 1/250 et 1/500 des plasmas à tester dans la solution D
- ajouter "en duplicate" 100 µl des différentes dilutions à tester (courbe de calibration et échantillon)

Pour préparer les blancs (ces échantillons ne comprennent pas de premier anticorps), il faut :

- ajouter également dans 4 puits 100 µl de la solution D "en duplicate"
- recouvrir la plaque d'un adhésif transparent et laisser incuber 1 h à 37 °C

*D-2 : Si l'échantillon à tester est un concentré d'immunoglobulines :*

Procéder comme décrit ci-dessus en préparant 250 µl d'une dilution du lot à 0,1; 0,05; 0,025 et 0,01 mg/ml dans le tampon D.

### E. Addition de l'antisérum de lapin marqué à la peroxydase (HRP) et spécifiquement dirigé contre les IgG humaines

- Laver 5 fois la plaque avec la solution C
- Additionner un deuxième anticorps qui est un antisérum de lapin anti-IgG humaines marqué à la peroxydase
  On peut notamment utiliser un anticorps vendu par la société Sigma sous le numéro de référence A8792 à une dilution de 1/60000 ou un anticorps vendu par la société Dako sous le numéro de référence P0214 à une dilution 1/28000.

- Ajouter dans chaque puits 100 µl d'antisérum de lapin et incuber 30 min à 37 °C.

F. Révélation des plaques

La révélation des plaques se fait par addition de substrat (TMB ou 3,3'-5,5'-Teramethylbenzidine liquide de Sigma (réf. : T8665)) amené à température ambiante.

- Laver 5 fois la plaque avec la solution C
- Ajouter 100 µl de solution TMB dans tous les puits
- Incuber pendant 15 min à 37 °C

G. Arrêt de la réaction

L'arrêt de la réaction s'effectue par addition de 100 µl de $H_2SO_4$ 0,5 N.

H. Mesure spectrophotométrique

Lire l'absorbance à 450 nm endéans les 15 min.

***Calcul du titre en anticorps anti-pneumocoques***

A. Etablissement d'une droite de calibration et titre en anticorps anti-pneumocoques

Par définition, une dilution d'un plasma pool standard à 1/500 possède un titre de 1000 mU/ml d'IgG anti-pneumocoques.
On porte en graphique l'absorbance nette :

$$Od_{net} = \text{Moyenne des } Od_{mesurées} - \text{Moyenne des } Od_{blancs}$$

obtenue pour chaque dilution de plasma pool en fonction du logarithme du titre d'anti-pneumocoques correspondant.
On détermine à l'aide d'un programme de régression linéaire l'équation de la droite de calibration.

B. Calcul du titre sur les échantillons inconnus

A l'aide de l'équation de la droite de calibration, on détermine le titre en mU/ml en anticorps anti-pneumocoques présents dans les échantillons inconnus en tenant compte des dilutions utilisées.
Le tableau 1 représente la valeur des densités obtenues à 450 nm.
Le titre de chacune des dilutions est recalculé en tenant compte du facteur de dilution.

Tableau 1

| Calibrateur | | O.D. Moy. | SDEV | O.D. Net (-Blc) |
|---|---|---|---|---|
| **Dilution** | **mU/ml** | | | |
| 1/125 | 4000 | 1,665 | 0,018 | 1,591 |
| 1/250 | 2000 | 1,172 | 0,039 | 1,098 |
| 1/500 | 1000 | 0,834 | 0,006 | 0,760 |
| 1/1000 | 500 | 0,536 | 0,010 | 0,462 |
| **Blanc** | | 0,074 | 0,011 | |

Le tableau 2 représente la valeur des densités obtenues en fonction des UI/ml (échelle logarithmique pour le calibrateur).

Tableau 2

| Calibrateur | |
|---|---|
| **Log mU/ml** | **O.D. Net** |
| 3,602 | 1,591 |
| 3,301 | 1,098 |
| 3,000 | 0,760 |
| 2,699 | 0,462 |

Les valeurs des densités optiques moyennes, corrigées du blanc, sont portées en graphique en fonction du titre (échelle semi-logarithmique).

Le titre des échantillons inconnus est calculé à l'aide des paramètres de la droite de calibration (figure 1), selon la méthode établie ci-dessous :

1) Equation de la droite de calibration : OD = a x (log mV/ml)-b ; avec a = 1, 007 et b = 2,26
2) Calcul du titre d'un échantillon inconnu : calculer la valeur du log (Titre). Log (Titre) = $(OD_{(mesurée)}-b)/a$
3) Titre (mV/ml) = $10^{log\ (Titre)}$
4) Calculer le titre moyen de l'échantillon aux deux différentes dilutions utilisées (1/250 et 1/500) sans oublier de tenir compte du facteur de dilution.

### Exemple 2 : Spécificité du test diagnostic vis-à-vis des antigènes capsulaires de *S. pneumoniae*

La réponse immunitaire dirigée contre les antigènes polysaccharidiques de *S. pneumoniae* se manifeste par la présence d'anticorps spécifiques de la paroi cellulaire et de la capsule (Sorensen et al., 1995 *Danish Medical Bulletin* 42, p. 47-53). Ces deux types d'anticorps peuvent être mis en évidence notamment chez les donneurs sains, les patients traités ou non par des immunoglobulines, ou vaccinés par lesdites immunoglobulines.

La détermination relative de ces deux types d'anticorps peut se révéler importante notamment pour les anticorps anti-antigéniques capsulaires dont le rôle protecteur contre une infection future a été démontré (Lee et al., 1997 *Critical Reviews in Microbiology* 23 (2), p. 121-142).

Par conséquent, le dispositif de diagnostic et/ou de quantification de l'invention peut être avantageusement utilisé pour assurer le suivi de telles infections.

Dans le but de distinguer les anticorps dirigés contre le polysaccharide C présent dans la paroi bactérienne et dans le vaccin anti-pneumocoque des anticorps capsulaires ; du polysaccharide C (purifié par exemple à partir de souches dépourvues de capsule ou obtenu par génie génétique) est utilisé dans une étape préliminaire pour neutraliser les anticorps spécifiquement dirigés contre ce dernier.

Une incubation préalable des échantillons à tester, par exemple en présence de 2,5μM/ml de polysaccharide C pendant une durée de 30 min. à 37°C, permet de doser les anticorps capsulaires dans les différentes préparations dans le test immunochimique en utilisant comme antigènes immobilisés des préparations d'antigènes polysaccharidiques (par exemple obtenus à partir du produit Pneumovax® ou à partir d'autres sources d'antigènes).

Dans ces conditions, le taux des anticorps capsulaires par rapport aux anticorps totaux est d'environ 50% dans les standards pool de plasma, de 80% dans le standard 89SF et de 10 à 90% chez les donneurs sains.

### Revendications

**1.**    Dispositif de détection et/ou de quantification du titre d'anticorps présents dans un liquide biologique d'un patient et spécifiques d'une réaction liée à une infection par un microorganisme, spécifiques d'une réaction auto-immunitaire ou spécifiques d'une allergie, **caractérisé en ce qu'**il comprend en tant que standard un échantillon biologique sélectionné à partir d'un pool de plasmas sanguins de plus de 200 individus sains.

**2.**    Dispositif selon la revendication 1, **caractérisé en ce que** l'échantillon biologique est sélectionné à partir d'un pool de plasmas sanguins de plus de 1000 individus sains.

**3.**    Dispositif selon la revendication 2, **caractérisé en ce que** l'échantillon biologique est sélectionné à partir d'un pool de plasmas sanguins de plus de 5000 individus sains.

**4.** Dispositif selon l'une quelconque des revendications précédentes, **caractérisé en ce qu'**il est une trousse de diagnostic et/ou de quantification.

**5.** Dispositif selon l'une quelconque des revendications précédentes, **caractérisé en ce que** ledit patient est un patient humain.

**6.** Dispositif selon l'une quelconque des revendications précédentes, **caractérisé en ce que** le microorganisme est un agent infectieux bactérien.

**7.** Dispositif selon la revendication 6, **caractérisé en ce que** l'agent infectieux bactérien est la souche *Streptococcus pneumoniae*.

**8.** Dispositif selon la revendication 6, **caractérisé en ce que** l'agent infectieux bactérien est une souche de *Neiseria.*

**9.** Dispositif selon la revendication 6, **caractérisé en ce que** l'agent infectieux bactérien est une souche d'*Hemophilus*.

**10.** Utilisation du dispositif selon l'une quelconque des revendications précédentes, pour la détection et/ou le suivi d'une infection par un microorganisme.

**11.** Utilisation selon la revendication 10, **caractérisée en ce que** la microorganisme est un agent infectieux bactérien choisi parmi le groupe constitué par les souches de *Streptococcus pneumoniae, Neiseria* et *Hemophilus.*

**Claims**

**1.** A device for detecting and/or quantifying the antibody titer present in a biological fluid of a patient and specific for a reaction linked to an infection with a microorganism, specific for an autoimmune reaction or specific for an allergy, **characterised in that** it comprises as standard a biological sample selected from a pool of blood plasmas from more than 200 healthy individuals.

**2.** The device according to claim 1, **characterised in that** the biological sample is selected from a pool of blood plasmas from more than 1000 healthy individuals.

**3.** The device according to claim 2, **characterised in that** the biological sample is selected from a pool of blood plasmas from more than 5000 healthy individuals.

**4.** The device according to any of the preceding claims, **characterised in that** it is a diagnostic and/or quantification kit.

**5.** The device according to any of the preceding claims, **characterised in that** said patient is a human patient.

**6.** The device according to any of the preceding claims, **characterised in that** the microorganism is a bacterial infectious agent.

**7.** The device according to claim 6, **characterised in that** the bacterial infectious agent is the strain *Streptococcus pneumoniae*.

**8.** The device according to claim 6, **characterised in that** the bacterial infectious agent is a strain of *Neiseria*.

**9.** The device according to claim 6, **characterised in that** the bacterial infectious agent is a strain of *Hemophilus*.

**10.** Use of the device according to any of the preceding claims, for detecting and/or monitoring an infection with a microorganism.

**11.** The use according to claim 10, **characterised in that** the microorganism is a bacterial infectious agent chosen from the group consisting of the strains of *Streptococcus pneumoniae, Neiseria* and *Hemophilus.*

**EP 1 008 854 B1**

**Patentansprüche**

1. Vorrichtung zur Erfassung und/oder Quantifizierung des Titers von Antikörpern, die in einer biologischen Flüssigkeit eines Patienten vorhanden und für eine Reaktion, die mit einer Infektion durch einen Mikroorganismus verbunden ist, für eine Autoimmunreaktion oder eine Allergie spezifisch sind, **dadurch gekennzeichnet, dass** sie als Standard ein biologisches Muster umfasst, das aus einem Blutplasmapool von mehr als 200 gesunden Individuen ausgewählt wird.

2. Vorrichtung nach Anspruch 1, **dadurch gekennzeichnet, dass** das biologische Muster aus einem Blutplasmapool von mehr als 1000 gesunden Individuen ausgewählt wird.

3. Vorrichtung nach Anspruch 2, **dadurch gekennzeichnet, dass** das biologische Muster aus einem Blutplasmapool von mehr als 5000 gesunden Individuen ausgewählt wird.

4. Vorrichtung nach einem der vorhergehenden Ansprüche, **dadurch gekennzeichnet, dass** es sich um ein Diagnose- und/oder Quantifizierungkit handelt.

5. Vorrichtung nach einem der vorhergehenden Ansprüche, **dadurch gekennzeichnet, dass** der Patient ein Mensch ist.

6. Vorrichtung nach einem der vorhergehenden Ansprüche, **dadurch gekennzeichnet, dass** der Mikroorganismus ein bakterieller Infektionserreger ist.

7. Vorrichtung nach Anspruch 6, **dadurch gekennzeichnet, dass** der bakterielle Infektionserreger der Stamm *Streptococcus pneumoniae* ist.

8. Vorrichtung nach Anspruch 6, **dadurch gekennzeichnet, dass** der bakterielle Infektionserreger ein Stamm von *Neiseria* ist.

9. Vorrichtung nach Anspruch 6, **dadurch gekennzeichnet, dass** der bakterielle Infektionserreger ein Stamm von *Hämophilus* ist.

10. Verwendung der Vorrichtung nach einem der vorhergehenden Ansprüche zur Erfassung und/oder Verlaufskontrolle einer Infektion durch einen Mikroorganismus.

11. Verwendung nach Anspruch 10, **dadurch gekennzeichnet, dass** der Mikroorganismus ein bakterieller Infektionserreger ist, der aus der Gruppe, die von den Stämmen *Streptococcus pneumoniae, Neiseria* und *Hämophilus* gebildet ist, ausgewählt wird.

FIG. 1